# EUROPEAN PATENT SPECIFICATION

(11) **EP 1 474 435 B1**
(45) Date of publication and mention of the grant of the patent: **22.02.2006**
(21) Application number: 03796075.4
(22) Date of filing: 02.12.2003
(51) Int. Cl.: C07J 5/00

(54) **PROCESS FOR THE PREPARATION OF 6-ALPHA FLUORINATED PREGNANES**
PROZESS ZUR HERSTELLUNG VON 6 ALPA-FLUORIERTEN PREGNANEN
PROCEDE DE PREPARATION DE DERIVES PREGNANES 6-ALPHA FLUORES

(30) Priority: 09.12.2002 EP 02027534
(43) Date of publication of application: 10.11.2004
(73) Proprietor: Sicor Inc., Irvine, CA 92618 (US)
(72) Inventor: MACDONALD, Peter, CH-6925 Gentilino (CH); ROSSETTO, Pierluigi, CH-6828 Balerna (CH)
(74) Representative: Pohlman, Sandra M.
(86) International application number: PCT/EP2003/050925
(87) International publication number: WO 2004/052911

(56) References cited:
- EP-A- 1 207 166
- WO-A-02/100878
- US-A- 5 478 957
- UMEMOTO T ET AL: "N-FLUOROPYRIDINIUM TRIFLATE AND ITS DERIVATIVES: USEFUL FLUORINATING AGENTS" TETRAHEDRON LETTERS, ELSEVIER SCIENCE PUBLISHERS, AMSTERDAM, NL, vol. 27, no. 37, 1986, pages 4465-4468, XP009004018 ISSN: 0040-4039

## Description

The present invention refers to an improved stereoselective process for the preparation of 6α-fluoropregnanes, comprising the fluorination with an electrophilic fluorination agent in the 6-position of selected pregnane derivatives in the presence of an amine salt of a strong add under substantially water-free reaction conditions.

Fluorosteroids represent useful antiinflammatory compounds and it is known that stereoisomers have different pharmacological efficiencies. It is also known that 6α-fluoropregnane derivatives have in general a higher efficiency than corresponding 6β-fluoro analogues. Several processes have been developed for obtaining 6α-fluoropregnanes, but all these processes suffer from poor stereoselectivity. There.is a need for a process giving a higher ratio of 6α- to 6β-stereoisomers for a more economic industrial-scale manufacture.

F. La Loggia et al. describe in US Patent Application Publication 2002/0062021 a process for the fluorination of pregnanes having the formula 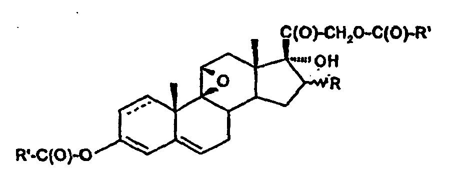 wherein R is chosen from H, OH, and an alkyl group with from 1 to 4 carbon atoms and R' is an alkyl group with from 1 to 4 carbon atoms, using electrophilic fluorination agents. Own investigations have shown that this process leads to reaction mixtures containing 6α/6β ratios of not higher than 90:10. Partial elimination of 6β-isomers could sometimes be obtained, but with an accompanying loss of product, by filtration of undissolved products from the reaction mixture. Pure 6α-fluoro derivatives can therefore only be obtained after further purification and I or isomerisation steps (see US-A-US 6,369,218), both of which inevitably lead to additional costs and significant losses of product.

J.-Y. Godard et al. disclose in US-A-5,478,957 a process for the introduction of a 6α-fluoro function into an *androstane* enol benzoate derivative (3-benzoyloxy-9β,11β-epoxy-16α-methyl-17β-methoxycarbonyl-Δ1,3,5-androstatrien-17α-ol) using an electrophilic fluorination agent, preferably Selectfluor®, in a water-containing solvent. We have found that the crude product (6α-fluoro-9β,11β-epoxy-16α-methyl-17β-methoxycarbonyl-ΔA1,4-androstadien-17α-ol-3-one) obtained in Stage D contains at least 4% of 6β-isomer, as well as significant amounts of 6-hydroxylated by-product. Application of the reaction conditions described in US 5,478,957 to an enol benzoate of a *pregnane* gave even more (6.5%) 6β-isomer formation, as well as a significant amount of 6-hydroxylated by-product. The purity of 6α-fluoropregnanes that could be obtained using the conditions of US 5,478,957 is thus unsatisfactory, and further purification would be necessary in order to obtain products suitable for pharmaceutical formulations. Even when such purifications are practicable, considerable amounts of the desired 6α-isomer are inevitably lost during the purification steps. It is also to be noted that, although the conversion of a pregnane into its corresponding androstane derivative through side-chain removal is relatively efficient (see, for example, Stage A of Example 1 in US 5,478,957), the reverse (reconstruction of the pregnane side-chain) constitutes a difficult multi-step operation. The products of US 5,478,957 are therefore not at all useful for obtaining valuable 6α-fluorinated pregnanes such as diflorasone, flumethasone, difluprednate, fluocinolone acetonide, fluocinonide, flunisolide, and others.

WO 02/100878 A1 describes a process for the preparation of flumethasone, where 9β,11-epoxy-16α-methyl-3,17,21-trihydroxy-pregna-1,3,5-triene-20-one-21-acetate-3-benzoate is used as an intermediate for fluorination in 6-position with an electrophilic fluorination agent.

It has surprisingly now been found that the stereoselectivity of electrophilic 6α-fluorinations of pregnanes can be greatly enhanced, and that the formation of side-products can be substantially suppressed, if 3-benzoyloxy- Δ3,5-pregnane derivatives are selected as substrates and the fluorination is carried out in a substantially water-free reaction mixture in the presence of a salt of a strong acid with a nitrogenous base. Under these conditions 6α/6β ratios of up to 99:1 can be obtained in reaction mixtures, with negligible formation of 6-hydroxylated, 4-fluorinated, or other by-products. Moreover, according to the fluorination process of the invention, higher chemical yields are obtained and, due to the high purity of the crude products, fewer purification operations are necessary in order to obtain useful pharmaceutical products.

One object of the invention is a process for the preparation of 6α-fluoro compounds of formula I, 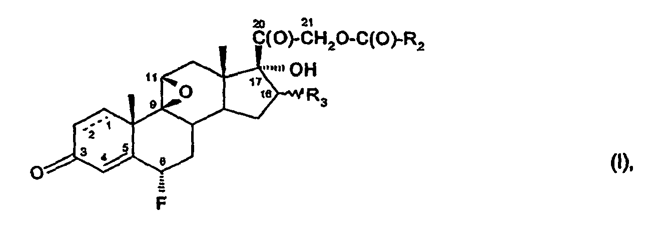
wherein
R₂ is hydrogen, C₁-C₈alkyl or C₃-C₈cycloalkyl; and
R₃ is hydrogen, C₁-C₈alkyl, or R₄-C(O)-O- where R₄ is C₁-C₈alkyl or C₁-C₈hydroxyalkyl;
comprising the fluorination of pregnane derivatives in the 6-position with an electrophilic fluorination agent, in an inert solvent and at ambient temperatures, characterised in that (1) a compound of formula II 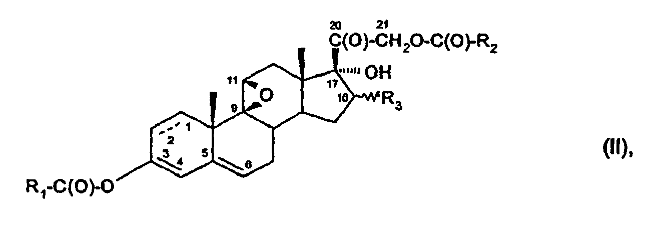
wherein
R₁ is phenyl or phenyl substituted with halogen, hydroxy, amino, mono- or di-C₁-C₈alkylamino, C₁-C₈alkyl, C₁-C₈alkoxy and/or C₁-C₈carbalkoxy; and R₂ and R₃ have the meanings given before;
is reacted with an electrophilic fluorination agent (2) in the presence of a salt of a strong acid with a nitrogenous base under (3) substantial water-free reaction conditions.

R₂ in formula I means preferably C₁-C₄alkyl and may be methyl, ethyl, n- or i-propyl, and the isomers of butyl, pentyl, hexyl, heptyl and octyl. R₂ in formula I as C₃-C₈cycloalkyl is preferably C₃-C₆cycloalkyl, and more preferably C₄-C₆Cycloalkyl. Cycloalkyl can be for example cyclopropyl, cyclobutyl, cyclopentyl, cyclohexyl, cycloheptyl and cyclooctyl. Preferred cycloalkyls are cyclopentyl and cyclohexyl.

R₂ in formula I is most preferably C₁-C₆alkyl and particularly preferred C₁-C₄alkyl.

R₃ is as alkyl preferably C₁-C₆alkyl, more preferably C₁-C₄alkyl, and mostly preferred C₁₋C₂alky. Examples for alkyl are methyl, ethyl, n- or i-propyl, and the isomers of butyl, pentyl, hexyl, heptyl and octyl. R₃ is as alkyl especially preferred methyl or ethyl.

R₄ in the residue R₄-C(O)-O- may have as alkyl the same preferred meanings as given before for R₃. R₄ is as alkyl mostly preferred methyl or ethyl. R₄ as hydroxyalkyl may contain 1 to 4 and more preferably 1 or 2 carbon atoms. Examples are hydroxymethyl and hydroxyethyl.

In a preferred embodiment, R₃ is selected from the group of hydrogen, methyl and acetyloxy,

inert solvents for this reaction are well known and may be selected from the group of polar and aprotic solvents. Examples are nitriles (acetonitril), N-dialkylated carboxylic acid amides (dimethyl formamide, diethyl formamide) or N-alkylated, cyclic carboxylic acid amides (N-methyl pyrrolidone, N-ethyl pyrrolidone), ethers (tetrahydrofurane, dioxane) and carboxylic esters (ethylacetate, methylbenzoate).

Ambient temperatures may mean a temperature range from -20 to 50 °C, preferably -10 to 40 °C, and most preferably 0 to 30 °C.

Preferred substituents for R₁ as phenyl are fluorine, chlorine, hydroxy, dimethylamino, methyl, ethyl, methoxy, ethoxy and methoxycarbonyl. Examples for substituted phenyl are 4-fluorophenyl, 2,4-difluorophenyl, 2,4,6-trfluorophenyl, 4-chlorophenyl, 2,4-dichlorophenyl, 2-or 4-hydroxyphenyl, 4-methylphenyl, 4-ethylphenyl, 2,4-dimethylphenyl, 2,4-diethylphenyl, 2,4,6-trimethylphenyl, 4-methoxyphenyl, 4-ethoxyphenyl, 2,4-dimethoxyphenyl, 2,4-diethoxyphenyl, 2,4,6-trimethoxyphenyl, 2-methyl-4-fluomphenyl, 2-methyl-4-chlorophenyl, 2- or 3- or 4-methoxycarbonylphenyl.

In a preferred embodiment R₁ is phenyl.

Suitable electrophilic fluorination agents are well known in the art and, in part, commercially available. These compounds include for example N-fluorosulfonamides, N-fluoropyridinium salts, N-fluorobis(trifluoromethanesulfonyl)imides, N-alkyl-N'-fluoro-1,4-diazoniabicyclo[2.2.2]octane salts, N-fluoro-N'-hydroxy-1,4-diazoniabicyclo[2.2.2]octane salts, and perchloryl fluoride.

Fluorinating agents are preferably selected from N-F quarternary salts due to their commercial availability and improved safety with respect to older reagants such as perchloryl fluoride. Examples of preferred fluorination agents are 1-chloromethyl-4-fluoro-1,4-diazoniabicyclo[2,2,2]octane-bistetrafluoroborate (Selectfluor®) and 1-fluoro-4-hydroxy1,4-diazoniabicyclo[2,2,2]octane-bistetrafluoroborate (Accufluor®).

The electrophilic fluorination agent may be used in excess without detriment, but since the excess reagent must be then destroyed after the reaction it is very desirable to avoid this additional process step. According to the process of the invention, substantially equimolar amounts of the fluorination agent and compounds of formula II are used and lead to completeness of the fluorination. Substantially equimolar amounts are therefore especially preferred. The molar ratio of compounds of formula II to fluorinating agent is preferably 1:1 to 1:0.95 and especially preferred is a molar ratio of 1:1.

Amine salts with an anion of a strong acid may correspond to formula III,

HB⁺ A⁻ (III),

wherein HB⁺ is the cation of an aliphatic, aromatic, cyclic aliphatic or cyclic aromatic nitrogenous base, and A⁻ is the anion of a strong organic or inorganic acid.

The cation HB⁺ may have more than one amine group, for example 1 to 4 or 1 to 2 amine groups and anions A⁻ are present in a number according to their positive charges.

The cation can be derived from ammonia, primary, secondary or tertiary amines, whereby tertiary amines are preferred. The amine contains preferably in total 1 to 24, more preferably 1 to 16 and mostly preferred 1 to 8 carbon atoms. The amines may contain 1 to 4, and more preferably one or two, nitrogen atoms.

The N-atoms of the amine may be substituted with C₁-C₁₂alkyl, preferably C₁-C₈akyl and most preferably C₁-C₄alkyl, C₃-C₈cycloalkyl,preferably C₅-C₈cycloalkyl, C₄-C₁₀heterocycloalkyl, preferably C₄-C₈heterocycloalkyl, C₈-C₁₀aryl, C₁-C₁₀aralkyl, C₅-C₁₀heteroaryl or C₅₋C₁₀heteroaralkyl. The N-atom of the amines can be part of an aliphatic or aromatic monocyclic or polycyclic aliphatic or aromatic ring (cyclic amines) and said N-atoms can be substituted with one residue as mentioned above. Alkyl groups at the N-atoms may be substituted, for example with C₃-C₈cycloalkyl, C₄-C₁₀heterocycloalkyl, C₁-C₈alkyl, C₁-C₈alkoxy or hydroxyl, cycloalkyl, heterocycloalkyl, aryl, heteroaryl, aralkyl and heteroaralkyl as well as rings of cyclic amines can be substituted for example with C₁-C₆alkyl, C₁-C₆alkoxy or hydroxyl.

Examples for amines, from which the cation HB⁺ is derived, are mono-, di- and preferably trialkylamines like methyl-, ethyl-, propyl-, butyl, -octyl-, dimethyl-, diethyl-, dipropyl-, dibutyl-, methyl-ethyl-, methyl-propyl-, methyl-butyl-, trimethyl-, triethyl-, tripropyl-, tributyl-, methyl-diethyl-, dimethyl-ethyl-amine. Other examples for amines, from which the cation A* is derived, are cycloalkyl-, heterocycloalkyl-, aryl-, aralkyl-, heteroaryl and heleroaraikyl-amines, preferably tertiary amines, like cyclohexyl-, cyclohexyl-methyl-, cyclohexyl-dimethyl-, tetrahydrofuranyl-, tetrahydrofuranyl-methyl, tetrahydrofuranyl-dimethyl-, phenyl-, phenyl-methyl-, phenyl-ethyl-, phenyl-butyl-, phenyl-dimethyl-, phenyl-diethyl-, benzyt-, benzyl-methyl-, benzyl-ethyl-, benzyl-isopropyl-, benzyl-butyl-, benzyl-dimethyl-, benzyl-diethyl-, furanyl-, furanylmethyl-, furanyl-dimethyl-, thiophenyl-, thiophenyl-methyl-, thiophenyl-dimethyl-, furanylmethyl-, furanylmethyl-dimethyl-amine. Examples for amines with substituted residues are ethanolamine, diethanolamine, triethanolamine, and N,N-dimethyl-ethanolamine.

Examples for aliphatic, cyclic or aromatic amines and for polyamines having more than one amino group are pyrrolidine, piperidine, N-methyl-pyrrolidine, N-methyl-piperidine, N-methylmorpholine, dimethylamino-N-methyl-piperidine, N,N' -diazabicycloheptane-, N,N'-diazabicydononane, 2H-pyrrole, imidazole, pyrazole, pyridine, pyrazine, pyrimidine, pyridazine, 3H-indole, 1H-indazole, purine isoquinoline, quinoline, phthalazine, naphthydrine, quinoxaline, quinazoline, pteridine, acridine, phenanthroline, phenazine, imidazoline, triazine, 2-piccoline, lutidine, benzimidazole, methylimidazole, pyrazole, 4-dimethylaminopyridine, 4-pyrrolidinopyridine, 1,3,5-triazine and 4-methylaminopyridine.

Preferred amines are selected from the group of cyclic, polycyclic and aromatic amines and aromatic amines are especially preferred. Preferred aromatic amines are pyridine and pyrimidine.

The anion may be derived from inorganic or organic acids and the anion is preferably selected from acids which do not cause side reactions like halogenations to avoid contamination of the desired product with impurities. Examples for anions of inorganic acids are halides, hydrogen sulphate, sulphate, mono- or di-hydrogen phosphate, and phosphate. Examples for anions of organic acids are carboxylates, sulfonates, phosphinates and phosphonates. The organic residues of the organic anions may be substituted, for example with halogen and especially fluorine, hydroxy, C₁-C₈alkyl or C₁-C₈alkoxy. Specific examples are formiate, acetate, trifluoracetate, oxalate, malonate, benzoate, fluorinated benzoates, methylsulfonate, trifluormethylsulfonate, phenylsulfonate, p-toluylsulfonate, fluorphenylsulfonate, methylphosphonate, phenylphosphonate. Especially preferred are sulfonates like methylsulfonates.

A particularly preferred amine salt is pyridine methylsulfonate.

The amount of amine salts can vary in a wide range and may be from 0.1 to 100 percent by weight, preferably 1 to 100 percent by weight, more preferably 5 to 100 percent by weight and mostly preferred 10 to 100 percent by weight, referred to the amount of compounds of formula II. It was found that a range of 50 to less than 100 percent by weight, for example 50 to 90 percent by weight, is useful in carrying out the process according to the invention.

The amine salts of formula III can be added to the reaction per se as pre-formed salts or the amine salts can be formed in situ in adding an amine and a strong acid to the reaction mixture, which is a preferred embodiment for carrying out the process according to the invention.

Substantial water-free reaction conditions in the context of the invention means that no water is added to the reaction mixture. Solvents and chemicals must not be dried and the presence of traces of water does not affect the reaction.

Another object of the invention are compounds of formula II as valuable intemiediates for the process according to the invention, 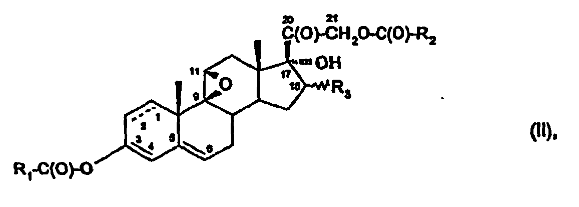
wherein R₁, R₂ and R₃ have the meanings given before, with the proviso that R₁ is not phenyl, when R₂ and R₃ both are methyl. R₁, R₂ and R₃ includes preferred embodiments given before.

Compounds of formula II are obtained in known manner and known or analogous processes through esterification or transesterification of 9,11β-epoxy-Δ4-pregnane-17β-ol-21-hydroxy-3,20-diones with carboxylic acids or derivatives thereof like carboxylic acid halides, anhydrides or esters. The preparation is advantageously carried out in two steps, since usually two different carboxylic acid residues must be introduced. In a first reaction step, the 21-hydroxy group can be selectively esterified for example with carboxylic acid anhydrides like acetic anhydride. In a second reaction step is formed the 3-enol ester with benzene carboxylic acid halides (bromides or chlorides). More details regarding this reaction are given in the examples. The compounds of formula II are obtained in high yields and purity and the crude reaction product can directly be used after separation from the reaction mixture for the fluorination according to the process of the instant invention. The crude reaction product can contain benzoic acid alkyl esters, which are formed during preparation through the addition of alkanols, for example methanol, ethanol, propanol or butanol. The amounts may be up to 30 percent by weight and preferably 0,1 to 20 percent by weight, referred to compounds of formula II. It may be advantageous to add said benzoic acid alkyl esters to the reaction mixture, when isolated and purified compounds of formula II are used in the fluorination process according to the invention.

The process of the invention may be carried out by dissolving or suspending compounds of formula 11 in a suitable solvent and cooling the solution to temperatures below 20 °C and preferably about 10 °C. The amine salt, or approximately equivalent amounts of an amine and a strong acid, are then added, followed by the addition of the electrophilic fluorination agent The fluorination is an exothermal reaction and care must be taken at this stage that the temperature does not exceed values which would effect and degrade compounds of formulae I and II. The fluorination agent is added preferably drop-wise or in portions and the reaction mixture is preferably cooled during this operation. The reaction mixture is stirred after the addition of the fluorination agent and the temperature may be increased to ambient values, preferably room temperature. The reaction progress can be controlled by the chromatographic determination of the starting material of formula I. The reaction is terminated when presence of the starting material can no longer be detected. The reaction time may be from 0.5 to 8 hours. The desired compounds of formula 11 can be isolated from the reaction mixture in known manner, for example in removing the solvent and filtering or extracting the resulting suspension, followed by re-crystallisation from a suitable solvent, for example from an alkanol.

The process according to the invention provides various advantages over prior art methods for the preparation of compounds of formula I, which are
a) a very high 6α/6β ratio of even higher than 99:1 in the crude reaction product;
b) reduced amounts of reaction by-products;
c) high chemical yields and short reaction times;
d) reduction of purification steps to obtain the desired 6α-fluorosteroid;
e) possibility of industrial scale manufacture;

The process according to the invention is very useful for the manufacture of fluorinated steorids, which are used as pharmaceutical effective compounds. Specific examples for such compounds are flumethasone, diflorasone, fluocinolone, difluprednate, and their derivatives.

The following examples illustrate the invention.

### A) Preparation of compounds of formula II

### Example A1: Preparation of 9β,11β-epoxy-16β-methyl-3,17,21-trihydroxy-pregna-1,3,5-triene-20-one-21-acetate-3-benzoate

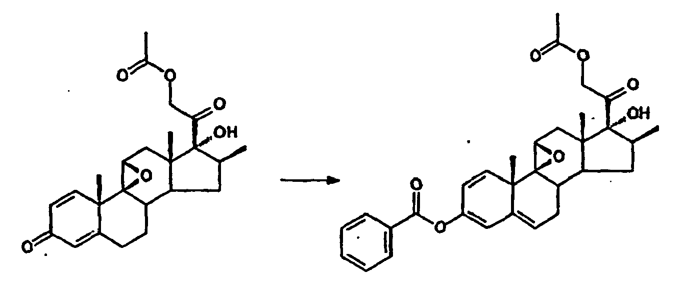

To a solution of 110 grams of 9β,11β-epoxy-17,21-dhydroxy-16β-methyl-pregna-1,4-diene-3,20-dione-21-acetate in 275 grams of pyridine at 75 °C in a nitrogen atmosphere are added 66 grams of benzoyl chloride. The reaction mixture is then held at said temperature for 180 minutes, cooled to 30 °C and diluted with 55 grams of methanol. After stirring for 30 minutes at 45 °C the solution is added to a cold mixture of 160 grams of 85% phosphoric acid. 1100 grams of water, and 1100 grams of dichloromethane. After stirring for 30 minutes the organic phase is separated and again washed with 1100 grams of water. After separation, the organic phase is diluted with 11 grams of pyridine and evaporated under reduced pressure to an oil consisting of the title compound, which is directly used in the subsequent step.
Trituration of the oily residue with diisopropyl ether gives the title compound as a pale tan crystalline powder.

### Example A2: Preparation of 9β,11β-epoxy-16α-methyl-3,17,21-trihydroxy-pregna-1,3,5-triene-20-one-21-acetate-3-benzoate

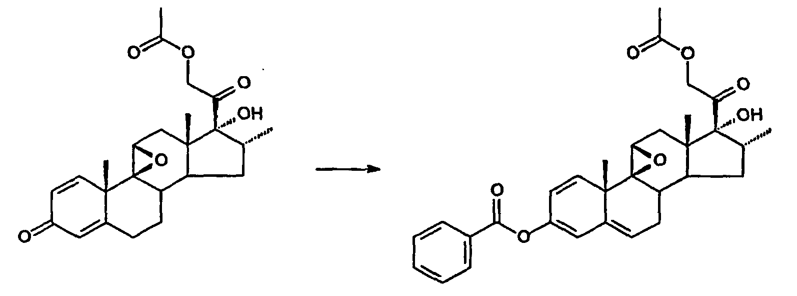

To a solution of 108 grams of 9β,11β-epoxy-17,21-dihydroxy-16α-methyl-pregna-1,4-diene-3,20-dione-21-acetate in 216 grams of pyridine at 75 °C in a nitrogen atmosphere are added 64.8 grams of benzoyl chloride. The reaction mixture is then held at said temperature for 180 minutes, cooled to 30 °C and diluted with 54 grams of methanol. After stirring for 30 minutes at 45°C the solution is added to a cold mixture of 126 grams of 85% phosphoric acid, 1080 grams of water, and 1080 grams of dichloromethane. After stirring for 30 minutes the organic phase is separated and again washed with 1080 grams of water. After separation, the organic phase is diluted with 10.8 grams of pyridine and evaporated under reduced pressure to an oil consisting of the title compound, which is directly used in the subsequent step.

### Example A3: Preparation of 9β,11β-epoxy-3,17,21-trihydroxy-pregna-1,3,5-triene-20-one-21-acetate-3-benzoate

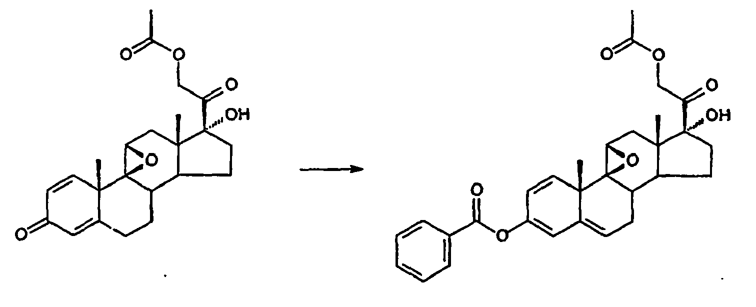

To a solution of 50 grams of 9β,11β-epoxy-17,21-dihydroxy-pregna-1,4-diene-3,20 dione-21-acetate in 135 grams of pyridine at 75 °C in a nitrogen atmosphere are added 30 grams of benzoyl chloride. The reaction mixture is then held at said temperature for 180 minutes, cooled to 30 °C and diluted with 25 grams of methanol. After stirring for 30 minutes at 45 °C the solution is added to a cold mixture of 78 grams of 85% phosphoric acid, 500 grams of water, and 500 grams of dichloromethane. After stirring for 30 minutes the organic phase is sparated and again washed with 500 grams of water. After separation, the organic phase is diluted with 5 grams of pyridine and evaporated under reduced pressure to a crystalline residue consisting of the title compound, which is directly used in the subsequent step.

### Example A4: Preparation of 9β,11β-epoxy-3,16α,17,21-tetrahydroxy-pregna-1,3,5-triene-20-one-16,21-diacetate-3-benzoate

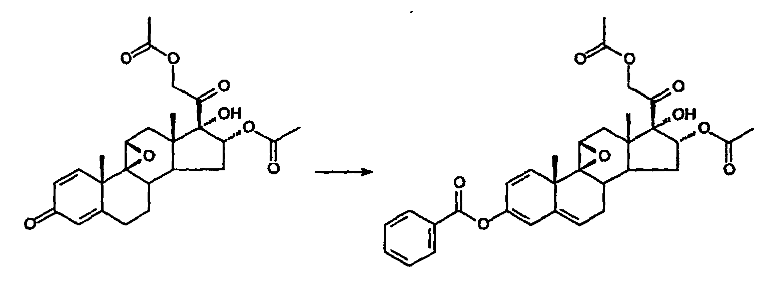

To a solution of 36 grams of 9β,11β-epoxy-16α,17,21-trihydroxy-pregna-1,4-diene-3,20-dione-16,21-diacetate in 72 grams of pyridine at 75°C in a nitrogen atmosphere are added 21.6 grams of benzoyl chloride. The reaction mixture is then held at said temperature for 180 minutes, cooled to 30°C and diluted with 18 grams of methanol. After stirring for 30 minutes at 45 °C the solution is added to a cold mixture of 42 grams of 85% phosphoric acid, 360 grams of water, and 360 grams of dichloromethane. After stirring for 30 minutes the organic phase is separated and again washed with 360 grams of water. After separation, the organic phase is diluted with 3.6 grams of pyridine and evaporated under reduced pressure to an oil consisting of the title compound, which is directly used in the subsequent step.

### Example A5: Preparation of 9β,11β-epoxy-3,17,21-trihydroxy-16α methyl-pregna-1,3,5-triene-20-one-3-benzoate-21-pivalate

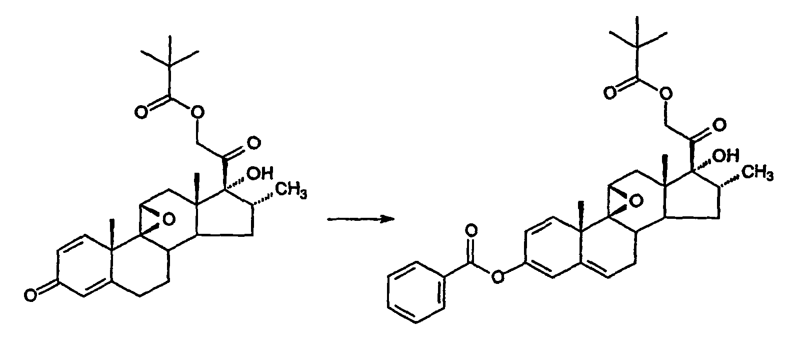

To a solution of 40 grams of 9β,11β-epoxy-17,21-dihydroxy-15α-methyl-pregna-1,4-diene-3,20-dione-21-pivalate in 100 grams of pyridine at 75°C in a nitrogen atmosphere are added 24 grams of benzoyl chloride. The reaction mixture is then held at said temperature for 300 minutes, cooled to 30 °C and diluted with 20 grams of methanol. After stirring for 30 minutes at 45 °C the solution is added to a cold mixture of 60 grams of 85% phosphoric acid, 400 grams of water, and 400 grams of dichloromethane. After stirring for 30 minutes the organic phase is separated and again washed with 400 grams of water. After separation, the organic phase is diluted with 4 grams of pyridine and evaporated under reduced pressure to an oil consisting of the title compound, which is directly used in the subsequent step

### Example A6: Preparation of 9β,11β-epoxy-16α-methyl-3,17,21-trihydroxy-pregna-1,3,5-triene-20-one-21 -acetate-3-toluate

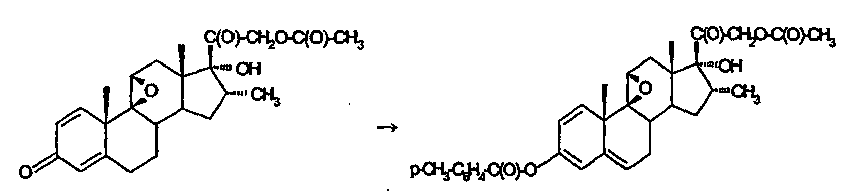

To a solution of 110 grams of 9β,11β-epoxy-17,21-dihydroxy-16α-methyl-pregna-1,4-diene-3,20-dione-21-acetate in 220 grams of pyridine at 75°C in a nitrogen atmosphere are added 64.8 grams of p-methyl benzoyl chloride. The reaction mixture is then held at said temperature for 240 minutes, cooled to 30 °C and diluted with 55 grams of methanol. After stirring for 30 minutes at 45°C the solution is added to a cold mixture of 129 grams of 85% phosphoric acid, 1100 grams of water, and 1100 grams of dichloromethane. After stirring for 30 minutes the organic phase is separated and again washed with 1100 grams of water. After separation, the organic phase is diluted with 11 grams of pyridine and evaporated under reduced pressure to an oil consisting of the title compound, which is directly used in the subsequent step.

### B) Preparation of compounds of formula I

### Example B1: Preparation of 9β,11β-epoxy-6α-fluoro-17,21-dihydroxy-16β-Methyl-pregna-1,4-diene-3,20-dione-21-acetate

To a solution of the oily residue according to Example A1 in 957 grams of acetonitrile at 0 °C are added 22 grams of pyridine and 25.5 grams of methanesulfonic acid, followed by 95.7 grams of Selectfluor® added at such a rate that the temperature of the mixture does not exceed 5 °C. The reaction mixture is then stirred at room temperature until HPLC analysis shows no starting compound remained. The HPLC analysis shows also that the resulting title compound contains only 1.0 % of 6β-epimer (6α/6β ratio is 99:1). The reaction mixture is diluted with 550 grams of water and then evoporated under reduced pressure to remove acetonitrile. Extraction of the resulting aqueous suspension using a mixture of dichloromethane and methanol (5:1 v/v) and subsequent crystallisation from methanol of the residue gives 91 grams of the pure title compound (HPLC analysis shows 0.69% of 6β-isomer).

### Example B2: Preparation of 9β, 11β-epoxy-6α-fluoro-17,21-dihydroxy-16α-methyl-pregna-1,4-diene-3,20-dione-21-acetate

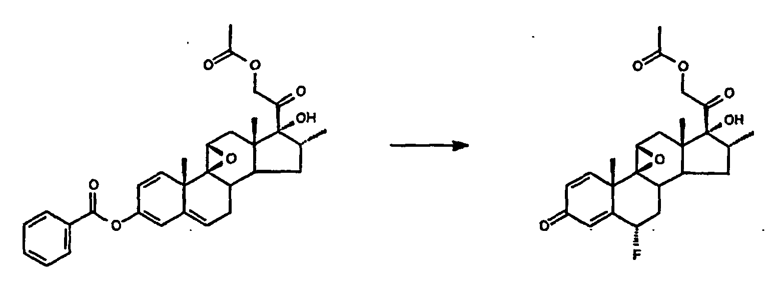

To a solution of the oily residue according to Example A2 in 940 grams of acetonitrile at 0° C are added 21.6 grams of pyridine and 25 grams of methanesulfonic acid, followed by 94 grams of Selectfluor® added at such a rate that the temperature of the mixture does not exceed 5°C. The reaction mixture is then stirred at room temperature until HPLC analysis shows no starting compound remained. HPLC analysis shows also that the resulting title compound contains only 1.0 % of 6β-epimer (6α/6β ratio is 99:1). The reaction mixture is diluted with 1080 grams of water and then evaporated under reduced pressure to remove acetonitrile. Extraction of the resulting aqueous suspension using a mixture of dichloromethane and methanol (5:1 v/v) and subsequent crystallisation from methanol of the residue gives 88 grams of the pure title compound.

### Example B3: Preparation of 9β,11β-epoxy-6α-fluoro-17,21-dihydroxy-pregna-1,4-diene-3,20-dione-21-acetate

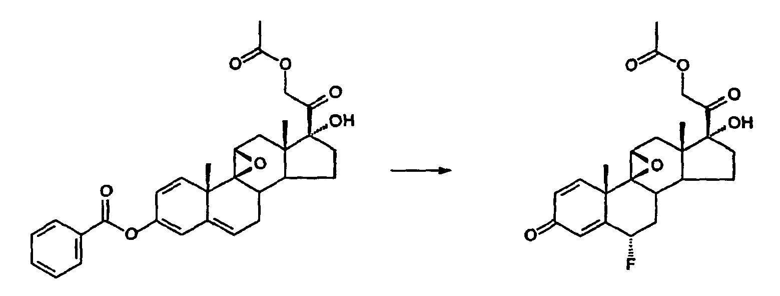

To a solution of the oily residue according to Example A3 in 400 grams of acetonitrile at 0 °C are added 10 grams of pyridine and 11.8 grams of methanesulfonic acid, followed by 44.5 grams of Selectfluor® added at such a rate that the temperature of the mixture does not exceed 5 °C. The reaction mixture is then stirred at room temperature until HPLC analysis shows no starting compound remained. HPLC analysis shows also that the resulting title compound contains less than 1.0 % of 6β-epimer. The reaction mixture is diluted with 500 grams of water and then evaporated under reduced pressure to remove acetonitrile. Extraction of the resulting aqueous suspension using a mixture of dichloromethane and methanol (4:1 v/v) and subsequent crystallisation from methanol of the residue gives 40.5 grams of the pure title compound (HPLC analysis shows 0.95% of 6β-isomer).

### Example B4: Preparation of 9β,11β-epoxy-6α-fluoro-16α.17.21-trihydroxy-pregna-1,4-diene-3,20-dione-16,21-acetate

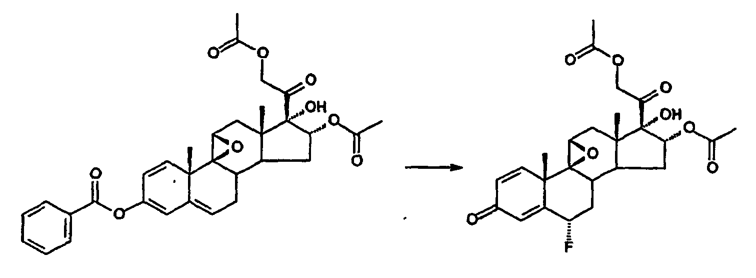

To a mixture of 4.61 grams of methanesulfonic acid in 156 grams of acetonitrile, 4 grams of pyridine, and 3 grams of methyl benzoate was added at 0-5° C 20 grams of crystalline 9β,11β-epoxy-3,16α,17,21-tetrahydroxy-pregna-1,3,5-triene-20-one-16,21-diacetate-3-benzoate obtained according to Example A4, followed by 12.6 grams of Selectfluor® added at such a rate that the temperature of the mixture does not exceed 5 °C. The reaction mixture is then stirred at room temperature until HPLC analysis shows no starting compound remained. HPLC analysis shows also that the resulting title compound contains only 1.2 % of 6β-epimer (6α/6β ratio is 99:1). The reaction mixture is diluted with 200 grams of water and then evaporated under reduced pressure to remove acetonitrile. Extraction of the resulting aqueous suspension using dichloromethane and subsequent crystallisation from diisopropyl ether of the residue gives 16.5 grams of the pure title compound (6β-epimer content 1.0%).

### Example B5: Preparation of 9β,11β-epoxy-6α-fluoro-17,21-dihydroxy-16α-methyl-pregna-1,4-diene-3,20-dione-21-pivalate

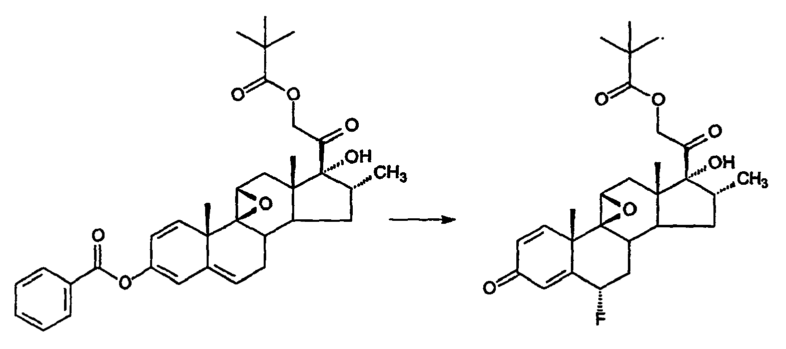

To a solution of the oily residue according to Example A5 in 400 grams of acetonitrile at 0 °C are added 4 grams of pyridine and 4.6 grams of methanesulfonic acid, followed by 31 grams of Selectfluor® added at such a rate that the temperature of the mixture does not exceed 5 °C. The reaction mixture is then stirred at room temperature until HPLC analysis shows no starting compound remained. HPLC analysis shows also that the resulting title compound contains only 1.7 % of 6β-epimer (6α/6β ratio is 98.3:1.7). The reaction mixture is diluted with 400 grams of water and then evaporated under reduced pressure to remove acetonitrile. Extraction of the resulting aqueous suspension using a mixture of dichloromethane and methanol (5:1 v/v) and subsequent crystallisation from methanol of the residue gives 29.5 grams of the pure title compound (HPLC analysis shows 0.35% of 6β-isomer).

### Example B6: Preparation of 9β,11β-epoxy-6α-fluoro-17,21-dihydroxy-16α-methyl-pregna-1,4-diene-3,20-dione-21-acetate

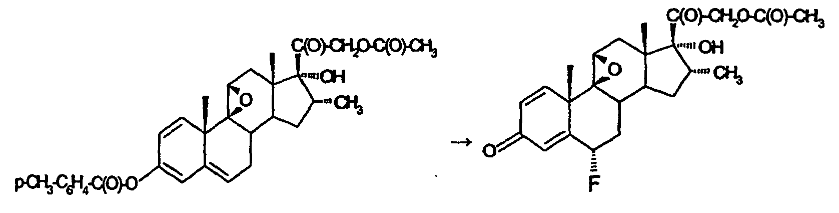

To a solution of the oily residue according to Example A6 in 960 grams of acetonitrile at 0° C are added 22 grams of pyridine and 25.5 grams of methanesulfonic acid, followed by 95.7 grams of Selectfluor® added at such a rate that the temperature of the mixture does not exceed 5 °C. The reaction mixture is then stirred at room temperature until HPLC analysis shows no starting compound remained. HPLC analysis shows also that the resulting title compound contains ca. 1.0 % of 6β-epimer (6α/6β ratio is 99:1). The reaction mixture is diluted with 1100 grams of water and then evaporated under reduced pressure to remove acetonitrile. Extraction of the resulting aqueous suspension using a mixture of dichloromethane and methanol (5:1 v/v) and subsequent crystallisation from methanol of the residue gives 87 grams of the pure title compound.

## Claims

1. A process for the preparation of 6α-fluoro compounds of formula 1, 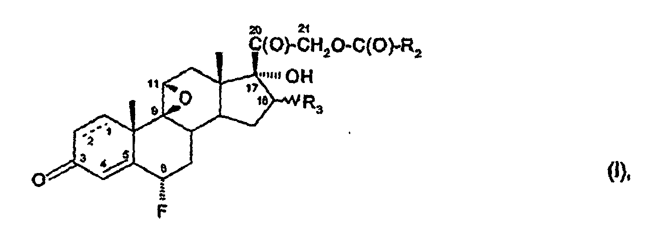
wherein
R₂ is hydrogen, C₁-C₈alkyl or C₃-C₈cycloalkyl; and
R₃ is hydrogen, C₁-C₆alkyl, or R₄-C(O)-O- where R₄ is C₁-C₈alkyl or C₁-C₈hydroxyalkyl;
comprising the fluorination of pregnane derivatives in the 6-position with an electrophilic fluorination agent, in an inert solvent and at ambient temperatures, **characterised in that** (1) a compound of formula II 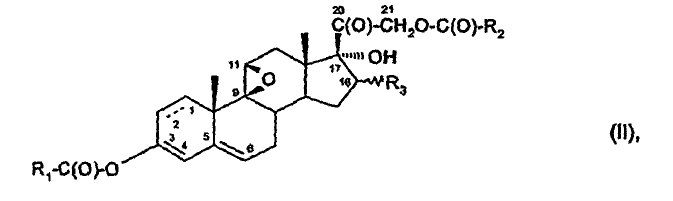
wherein
R₁ is phenyl or phenyl substituted with halogen, hydroxy, amino, mono- or di-C₁-C₈alkylamino, C₁-C₈alkyl, C₁-C₈alkoxy and/or C₁-C₈carbalkoxy; and R₂ and R₃ have the meanings given before;
is reacted with an electrophilic fluorination agent (2) in the presence of a salt of a strong acid with a nitrogenous base under (3) substantial water-free reaction conditions.

2. A process according to claim 1, wherein R₂ is methyl.

3. A process according to daims 1 or 2, wherein R₃ is hydrogen, methyl or acetoxy.

4. A process according to claims 1 to 3, wherein R₁ is phenyl or phenyl substituted with fluorine, chlorine, hydroxy, dimethylamino, methyl, ethyl, methoxy, ethoxy and methoxycarbonyl.

5. A process according to claim 1, wherein the solvent is selected from the group of nitriles, N-dialkylated carboxylic acid amides or N-alkylated cyclic carboxylic acid amides, ethers and carboxylic esters.

6. A process according to claim 1, wherein the reaction temperature is from -20 °C to 50 °C.

7. A process according to claim 1, wherein the fluorinating agent is 1-chloromethyl-4-fiuoro-1,4-diazoniabicyclo[2,2.2]octan-bistetrafluoroborate, or 1-fluoro-4-hydroxy1,4-diazoniabicyclo[2,2,2]octane-bistetrafluoroborate.

8. A process according to claim 1, wherein the amine salt corresponds to formula III,
HB⁺A⁻ (III),
wherein HB⁺ is the cation of an aliphatic, aromatic, cyclic aliphatic or cyclic aromatic nitrogen base, and A⁻ is the anion of a strong organic or inorganic acid, and wherein the amine salt is preferably pyridine methylsulfonate.

9. A process according to claim 1, wherein the amount of amine salt is from 0.1 to 100 and preferably 50 to 90 percent by weight, referred to the amount of compounds of formula II.

10. Compounds of formula II, 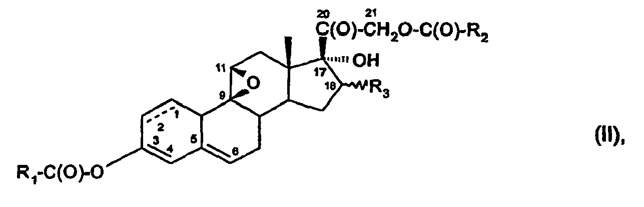
wherein R₁, R₂ and R₃ have the meanings given in claim 1, with the proviso that R₁ is not phenyl, when R₂ and R₃ are methyl.

## Patentansprüche

1. Ein Verfahren zur Herstellung von 6α-Fluoro Verbindungen der Strukturformel I, 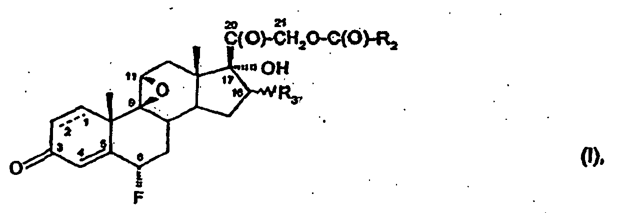
worin
R₂ Wasserstoff, C₁-C₈ Alkyl oder C₃-C₈ Cycloalkyl; und
R₃ Wasserstoff, C₁-C₈ Alkyl oder R₄-C(O)-O-, wobei R₄ C₁-C₈ Alkyl oder C₁-C₈ Hydroxyalkyl ist, bedeuten,
welcher die Fluorierung von Pregnan-Derivativen in 6-Position mit einem elektrophilen Fluorierungsmittel, in einem inerten Loesungsmittel und bei Umgebungstemperatur beinhaltet, **dadurch gekennzeichnet, dass** (1) eine Verbindung der Strukturformel II, 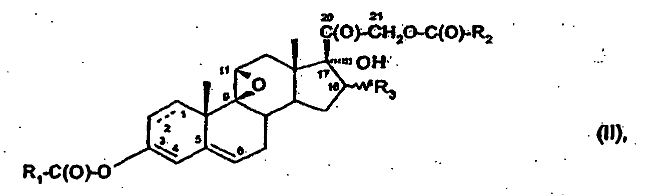
worin
R₁ Phenyl oder Phenyl, das mit Halogen, Amino, Mono- oder Di-C₁-C₈ Alkylamino, C₁-C₈ Alkyl, C₁-C₈ Alkoxy und/oder C₁-C₈ Carbalkoxy substituiert ist, darstellt; und R₂ und R₃ die vorher angegebenen Bedeutungen haben, mit einem elektrophilen Fluorierungsmittel (2) in Anwesenheit eines Salzes einer starken Säure mit einer stickstoffhaltigen Base (3) unter weitgehend wasserfreien Bedingungen umgesetzt wird.

2. Ein Verfahren gemäß Anspruch 1, worin R₁ Methyl bedeutet.

3. Ein Verfahren gemäß den Ansprüchen 1 oder 2, worin R₃ Wasserstoff Methyl oder Acetoxy bedeutet.

4. Ein Verfahren gemäß Ansprüchen 1 bis 3, worin R₁ Phenyl oder Phenyl, das mit Fluor, Chlor, Hydroxy, Dimethylamino, Methyl, Ethyl, Methoxy, Ethoxy und Methoxycarbonyl substituiert ist, bedeutet.

5. Ein Verfahren gemäß Anspruch 1, wobei das Lösungsmittel ausgewählt ist aus der Gruppe der Nitrile, N-dialkylierten Carbonsäureamiden oder N-alkylierten cyclischen Carbonsäureamiden, Ethern und Carbonsäurestern.

6. Ein Verfahren gemäß Anspruch 1, wobei die Reaktionstemperatur von -20°C bis 50°C ist.

7. Ein Verfahren gemäß Anspruch 1, worin das Fluorierungsmittel 1-Chloromethyl-4-fluoro-1,4-diazoniabicyclo[2,2,2]octan-bistetrafluoroborat oder 1-Fluoro-4-hydroxy-1,4-diazoniabicyclo[2,2,2]octan-bistetrafluoroborat ist.

8. Ein Verfahren gemäß Anspruch 1,worin das Aminosalz der Formel III,
HB⁺A⁻ (III),
entspricht, worin HB⁺ das Kation einer aliphatischen, aromatischen, cyclisch aliphatischen oder cyclisch aromatischen stickstoffhaltigen Base, und A⁻ ein Anion einer starken organischen oder anorganischen Säure ist, und worin das Aminosalz vorzugsweise Pyridinmethylsulfonat ist.

9. Ein Verfahren gemäß Anspruch 1, worin die Menge des Aminosalzes 0.1 bis 100, vorzugsweise 50 bis 90 Gewichtsprozent bezogen auf die Menge der Verbindung der Formel II ist.

10. Verbindungen der Formel II, 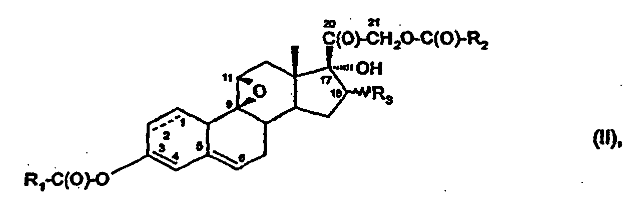
worin R₁, R₂ und R₃ wie in Anspruch 1 definiert sind, unter der Bedingung, dass R₁ nicht Phenyl bedeutet, wenn R₂ und R₃ Methyl bedeuten.

## Revendications

1. Procédé pour la préparation de composés à fonction 6α-fluoro, de formule I, 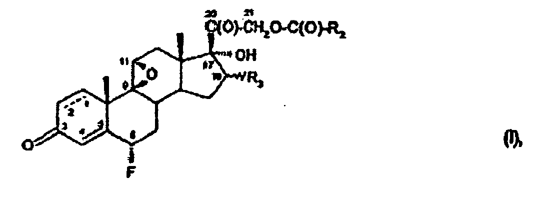
dans laquelle
R₂ représente l'hydrogène, un groupe alkyle en C₁ à C₈ ou cycloalkyle en C₃ à C₈ ; et
R₃ représente l'hydrogène, un groupe alkyle en C₁ à C₈ ou R₄-C(O)-O- dans lequel R₄ représente un groupe alkyle en C₁ à C₈ ou hydroxyalkyle en C₁ à C₈ ;
comprenant la fluoration de dérivés de prégnane en position 6 avec un agent de fluoration électrophile, dans un solvant inerte et aux températures ambiantes, **caractérisé en ce que** (1) un composé de formule II 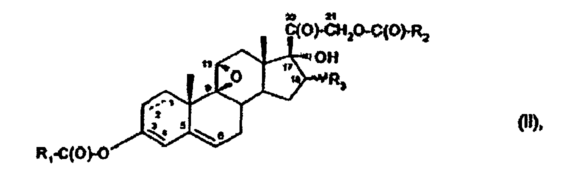
dans laquelle
R₁ représente un groupe phényle ou phényle substitué avec un substituant halogéno, hydroxy, amino, mono- ou di (alkyle en C₁ à C₈) amino, alkyle en C₁ à C₈, alkoxy en C₁ à C₈ et/ou carbalkoxy en C₁ à C₈ ; et R₂ et R₃ répondent aux définitions précitées ;
est amené à réagir avec un agent de fluoration électrophile (2) en présence d'un sel d'un acide fort avec une base azotée dans (3) des conditions réactionnelles pratiquement anhydres.

2. Procédé suivant la revendication 1, dans lequel R₂ représente un groupe méthyle.

3. Procédé suivant la revendication 1 ou 2, dans lequel R₃ représente l'hydrogène, un groupe méthyle ou acétoxy.

4. Procédé suivant les revendications 1 à 3, dans lequel R₁ représente un groupe phényle ou phényle substitué avec le fluor, le chlore, des groupes hydroxy, diméthylamino, méthyle, éthyle, méthoxy, éthoxy, et méthoxycarbonyle.

5. Procédé suivant la revendication 1, dans lequel le solvant est choisi dans le groupe comprenant des nitriles, des amides d'acides carboxyliques N-dialkylés ou des amides d'acides carboxyliques cycliques N-alkylés, des éthers et des esters carboxyliques.

6. Procédé suivant la revendication 1, dans lequel la température réactionnelle est comprise dans l'intervalle de -20°C à 50°C.

7. Procédé suivant la revendication 1, dans lequel l'agent de fluoration est le bis-tétrafluoroborate de 1-chlorométhyl-4-fluoro-1,4-diazoniabicyclo [2,2,2]octane ou le bis-tétrafluoroborate de 1-fluoro-4-hydroxy-1,4-diazoniabicyclo-[2,2,2]octane.

8. Procédé suivant la revendication 1, dans lequel le sel d'amine correspond à la formule III
HB⁺A⁻ (III),
dans laquelle HB⁺ représente le cation d'une base azotée aliphatique, aromatique, aliphatique cyclique ou aromatique cyclique et A⁻ représente l'anion d'un acide organique ou inorganique fort, et dans lequel le sel d'amine est de préférence le méthylsulfonate de pyridine.

9. Procédé suivant la revendication 1, dans lequel la quantité de sel d'amine est comprise dans l'intervalle de 0,1 à 100 et de préférence de 50 à 90 % en poids, par rapport à la quantité de composés de formule II.

10. Composés de formule II 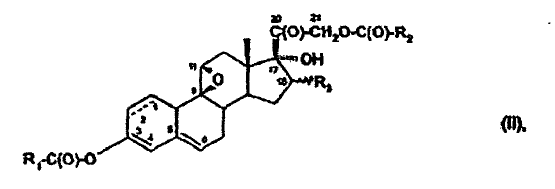
dans laquelle R₁, R₂ et R₃ répondent aux définitions indiquées dans la revendication 1, sous réserve que R₁ ne représente pas un groupe phényle, lorsque R₂ et R₃ représentent des groupes méthyle.
